Europäisches Patentamt

European Patent Office

Office européen des brevets

(19)

(11) Publication number: **0 113 215**
**B1**

(12) **EUROPEAN PATENT SPECIFICATION**

(45) Date of publication of patent specification: 21.09.88

(51) Int. Cl.⁴: **C 12 P 7/56,** C 12 N 1/20 //
C12R1/225

(21) Application number: 83307542.7

(22) Date of filing: 12.12.83

(54) **Microorganisms of the species Lactobacillus italicus, processes for their preparation and their use in the production of lactic acid.**

(30) Priority: 11.12.82 JP 217653/82

(43) Date of publication of application:
11.07.84 Bulletin 84/28

(45) Publication of the grant of the patent:
21.09.88 Bulletin 88/38

(84) Designated Contracting States:
DE FR GB

(56) References cited:
WO-A-80/02282
FR-A-2 126 452
GB-A- 936 339

(73) Proprietor: KYOWA HAKKO KOGYO KABUSHIKI
KAISHA
6-1, Ohte-machi 1-chome
Chiyoda-ku Tokyo 100 (JP)

(72) Inventor: Kenichiro, Takayama
1-9-10, Tobio
Atsugi-shi Kanagawa-ken (JP)
Inventor: Isumi, Masunaga
1-13-6, Mita Tama-ku
Kawasaki-shi Kanagawa-ken (JP)

(74) Representative: Watkins, Arnold Jack et al
European Patent Attorney Frank B. Dehn & Co.
Imperial House 15-19 Kingsway
London WC2B 6UZ (GB)

Courier Press, Leamington Spa, England.

## Description

The present invention relates to microorganisms of the species *Lactobacillus italicus*, processes for their preparation and their use in the production of lactic acid. The lactobacilli may be isolated from a dough for making bread.

*Lactobacillus sanfrancisco* is a known microorganism isolated from a dough for sour dough French bread conventionally made in the San Francisco Bay area of the United States, and is useful in the preparation of a liquid starter and dough for sour dough bakery products such as sour dough French bread (FR—A—2126452, and its equivalent US 3891773, and US 3 734 743). This microorganism is capable of growing in the presence of a sour dough yeast, viz. *Torulopsis holmii*.

As a result of various studies, we have isolated certain microorganisms from a dough used for the preparation of panettone which is a type of Italian rich bread. The present invention is based upon the discovery that the microorganisms which we have isolated are capable of growing in the absence of yeast and are of potential interest as lactic acid-producing microorganisms.

According to one feature of the present invention, there is provided a microorganism of the species *Lactobacillus italicus* having the following taxonomic characteristics:

(1) Gram-positive rods in a size of 0.8—1.0×2—7 μ;
(2) formation of spore: negative;
(3) motility: negative;
(4) anaerobic to facultative aerobic;
(5) growth temperature: 15—38°C, optimal 28—32°C;
(6) growth pH: 4.2—6.5, optimal 5.4—5.8;
(7) catalase: negative; and
(8) capable of assimilating glucose and maltose to produce DL-lactic acid, ethyl alcohol and carbon dioxide but not capable of assimilating arabinose, xylose, galactose, acetic acid, citric acid, gluconic acid or propionic acid as the sole source of carbon;
(9) DL-lactic acid and ethyl alcohol are produced from glucose or maltose at an approximate ratio of 1:0.4—0.6; and
(10) capable of growing in the absence of yeast; in or on a sterile medium.

The microorganisms in one embodiment may be further characterised in that glucose and maltose are the sole assimilable carbon sources.

The microorganisms of the present invention have been designated by us as *Lactobacillus italicus* for reasons hereinafter described and include the following two strains:

(I) *Lactobacillus italicus* TL-1 (FERM-BP 189) and
(II) *Lactobacillus italicus* SH-150 (FERM-BP 190).

The above-mentioned two microorganisms were deposited with the Fermentation Research Institute Agency of Industrial Science and Technology, Ministry of International Trade and Industry of 1—3, Higashi 1-chome, Yatabe-machi Tsukuba-gun, Ibaraki-ken 305, Japan under the Budapest Treaty on 29th September 1982.

The taxonomic characteristics of these two microorganisms are identical with those of various known lactobacilli such as, for example, *Lactobacillus sanfrancisco* except as specified below:

(I) *L. italicus* TL-1 (FERM-BP 189):
A. Morphological characteristics:
Where this strain is cultured in a liquid medium such as, for example, an ML medium as hereinafter defined, the present strain appears as rods having a size of (0.8—1.0)×(2—7) μ, and occurs in the form of a single rod or in chains. This strain is non-motile, non-sporulating and Gram-positive.

Composition of ML medium: peptine 12.5 g; yeast extract 12.5 g; potassium monohydrogenphosphate 250 mg; potassium dihydrogenphosphate 250 mg; magnesium sulfate 100 mg; manganese sulfate 5.0 mg; ferrous sulfate 5.0 mg; cysteine hydrochloride 300 mg; Tween 80® (commercial product of Atlas Chemical Industry, Inc., U.S.A.). 300 mg; sodium acetate 10 g; glucose 10 g; maltose 10 g; water 1000 ml (adjusted to a pH of 5.6 with 1N or 6N-HCl).

B. Cultural characteristics:
(1) ML agar plate:
Where this strain is cultured on an ML agar plate medium prepared by adding 20 g of agar to ML medium at 30°C for 2 days, round, smooth, entire, raised, semi-transparent and milky white colonies are formed, the diameter of the colonies being not greater than about 1 mm.

(2) ML broth:
The culture exhibits a moderate turbidity. No growth on the surface layer. Flocculent sediment is formed.

(3) ML agar stab:
Growth is observed along with the stab and on the surface layer.

C. Physiological characteristics:
(1) Growth temperature: range 15—38°C. Optimal 28—32°C.
(2) Growth pH: range 4.2 to 6.5. Optimal 5.4 to 5.8.
(3) Relation to oxygen:
Anaerobic to facultative aerobic. Better growth in an atmosphere of carbon dioxide, even though growth is possible under aerobic conditions.

(4) Required substances for the growth:
An unsaturated fatty acid is not required for growth on an ML medium.

(5) Production of acid and gas from sugars:
Produced from glucose and maltose, and not produced from arabinose, xylose, ribose, fructose, mannitol, galactose, sucrose, lactose, melibiose and salicin.

(6) Assimilation of carbon sources:
Maltose and glucose are assimilable, although maltose is more strongly assimilated than glucose.
Arabinose, xylose, galactose, acetic acid, citric acid, gluconic acid and propionic acid are not assimilated as sole carbon sources.

(7) Liquefaction of gelatin: negative.
(8) Litmus milk: unchanged.
(9) Reduction of nitrate: negative.
(10) Denitrification: negative.
(11) Urease: negative.
(12) Catalase: negative.
(13) Oxidase: negative.
(14) Formation of indole: negative.
(15) Formation of hydrogen sulfide: negative.
(16) Hydrolysis of starch: negative.
(17) VP reaction: negative.
(18) DL-lactic acid and ethyl alcohol are produced from glucose or maltose at an approximate ratio of 1:0.4—0.6.

(II) *Lactobacillus italicus* SH-150 (FERM-BP 190):
Similar taxonomic characteristics to those of *L. italicus* TL-1 (FERM-BP 189) except for the requirement of an unsaturated fatty acid for growth.
As hereinbefore described, *L. italicus* TL-1 (FERM-BP 189) and *L. italicus* SH-150 (FERM-BP 190) are Gram-positive rods which are non-sporulating, non-motile, anaerobic to facultative aerobic and catalase negative. Moreover, they are capable of producing lactic acid and ethyl alcohol from glucose and maltose. Thus, it is apparent that they are classified into the genus *Lactobacillus*. However, the present strains differ from all *lactobacilli* disclosed, for example, in Bergey's Manual of Determinative Bacteriology, 8th Edition (1974) in so far as the following identifying characteristics are concerned:—
(a) they decompose glucose and maltose, but no other carbohydrate whilst various other known lactobacilli decompose many carbohydrates;
(b) the optimal growth pH is from 5.4 to 5.8, whilst various known lactobacilli exhibit an optimal pH of from 6.5 to 7.0;
(c) they are incapable of assimilating gluconate, whilst various other lactobacilli of the heterofermentative type are capable of decomposing gluconate;
(d) the present strains differ from *Lactobacillus sanfrancisco* which is only capable of metabolizing glucose with difficulty and produces lactic acid and acetic acid from maltose. Moreover, a fresh yeast extract is required for the growth of *L. sanfrancisco*.
For these reasons, it is believed that the microorganisms of the present invention designated by us as *Lactobacillus italicus* are new microorganisms.
The taxonomic characteristics of the present strains were determined by the methods described in "Classification and Identification of Microorganisms" [edited by Takeshi Hasegawa and published by Gakkai Shuppan Centre Tokyo (1975)] and Bergey's Manual of Determinative Bacteriology, 8th Edition (1974) referred to above. With regard to *Lactobacillus sanfrancisco*, reference was made to Applied Microbiology, *21*, 459—465 (1971).
According to a further feature of the present invention there is provided a process for the preparation of a microorganism as hereinbefore defined which comprises inoculating an agar medium with an aqueous suspension of panettone dough, incubating the inoculated medium under anaerobic conditions to form colonies on said medium, isolating *Lactobacillus italicus* having the identifying characteristics hereinbefore defined and culturing the isolated strain(s) in or on a sterile medium.

Panettone dough is described in Bakers Digest October 76 80 (1977).

*L. italicus* of the present invention may be cultured, for example, in the following manner:—

Various solid and liquid media may be used for culturing *L. italicus* in so far as they are suitable for culturing lactobacilli and contain glucose and maltose as carbon sources. As carbon sources, various naturally occurring substances containing glucose and maltose such as, for example, starch hydrolyzate liquor, wheat flour and the like may be used. If desired, various organic acids such as, for example, acetic acid, citric acid and the like may be added to the medium in order to promote the growth of *L. italicus*. The use of various materials containing organic nitrogen such as, for example, yeast extract, peptone, meat extract, casein, corn steep liquor, tomato juice and the like as nitrogen sources is advantageous for promoting the growth of *L. italicus*, and thus it is preferred to add to the medium inorganic compounds such as, for example, ammonium sulfate, ammonium nitrate, ammonium phosphate and the like. It is also advantageous to add to the medium, for example, phosphoric acid, various ions of metals such as e.g. potassium, sodium, magnesium, manganese, iron, molybdenum and the like. The addition of, for example, cysteine, cystine, glutathione, ascorbic acid and the like to the medium is also advantageous in order to reduce the partial pressure of oxygen. Where an unsaturated fatty acid is required for the growth of the cultured microorganism, it is necessary to add a suitable agent such as, for example, oleic acid, Tween 80® (a surface active agent, commercially available from Atlas Chemical Industries, Inc., U.S.A.), lecithin and the like to the medium.

The culturing may for example be effected at a temperature of from 15 to 38°C, preferably from 28 to 32°C and at a pH of from 4.2—6.5, especially from 5.4 to 5.8 for 1 to 2 days. As the present strains are more or less anaerobic, the culturing may preferably be effected under anaerobic conditions in the presence of carbon dioxide.

In such a case, the mixing ratio of carbon dioxide with air may be from 25 to 95% by volume.

Especially good results may be obtained, for example, by using an ML medium as hereinbefore defined in order to isolate the present strain from a dough used for the preparation of panettone as hereinbefore described. Thus, for example, a panettone dough is suspended in a sterilized physiological saline solution, suitable buffer solution and the like and the mixture is smeared, for example, on an ML agar plate medium as hereinbefore defined for culturing at a temperature of from 28 to 32°C for 1—2 days under anaerobic conditions to obtain a pure culture of *L. italicus* of the present invention.

According to a further feature of the present invention there is provided a process for the preparation of lactic acid which comprises culturing a microorganism as hereinbefore defined in or on a medium therefor whereby to obtain a cultured liquor containing lactic acid. The lactic acid obtained is preferably isolated from the obtained cultured liquor.

The following non-limiting Examples illustrate the present invention.

Example 1

A panettone dough purchased from the Italian market (0.3 g) was suspended in a physiological saline solution (10 ml) under sterilized conditions. The suspension thus-prepared was multiply diluted with the same solution. Then, on each occasion, the diluted dough suspension (0.1 ml) was smeared on an ML medium as hereinbefore defined, and a Gas Pak System (Commercial Product of Baltimore Biological Laboratories, Inc., U.S.A.) was used to provide an atmosphere of carbon dioxide, in which the culturing was effected at a temperature of 30°C for 2 days to obtain semi-transparent, milky white colonies having a diameter of not greater than about 1 mm. The colonies were picked out and transferred to another ML agar plate medium for further culturing under similar conditions for 1—2 days. In this manner, 179 strains in total were isolated from 2 kinds of panettone dough, which were very similar to each other. Even though very small differences were noted between them, it was noted that all strains belonged to the same species. In view of the differences in the unsaturated fatty acid requirement for growth, *Lactobacillus italicus* TL-1 (FERM-BP 189) (negative requirement) and *L. italicus* SH-150 (FERM-BP 190) (positive requirement) were selected. In these cases, the number of the microorganisms present in the panettone doughs used was about $7 \times 10^8$ cell per gram.

Example 2

A medium containing yeast extract (3 g/l), peptone (5 g/l), $KH_2PO_4$ (0.25 g/l), $MgSO_4 \cdot 7H_2O$ (0.1 g/l), $MnSO_4 \cdot 4H_2O$ (5.0 mg/l), $FeSO_4 \cdot 7H_2O$ (5.0 mg/l), cysteine HCl (300 mg/l) and Tween 80® (300 mg/l) and having an initial pH adjusted to 5.6 with acetic acid, was used as a basal medium, from which 6 types of media were prepared by adding to the basal medium respectively the additives containing carbon sources shown in Table 1. On each occasion, the thus-prepared medium was put into a large test tube and was sterilized at a temperature of 120°C for 15 minutes in an autoclave. Each medium was then inoculated with *L. italicus* TL-1 (FERM-BP 189) or *L. italicus* SH-150 (FERM-BP 190) (about $10^8$ cells per ml) obtained from the ML agar plate medium hereinbefore described and incubated at a temperature of 30°C for the period shown in Table 1. The resultant growth shown in Table 1 is expressed by the turbidity of the cultured liquor, which was measured by the optical density at 660 m$\mu$.

4

TABLE 1

*L. italicus*

| Additive | (hours) | TL-1 (FERM-BP 189) | | SH-150 (FERM-BP 190) | |
|---|---|---|---|---|---|
| | | 24 | 60 | 24 | 60 |
| 1. No additive | | 0.03 | 0.05 | 0.03 | 0.04 |
| 2. Glucose 10 g/l | | 0.41 | 1.06 | 0.11 | 1.14 |
| 3. Maltose 10 g/l | | 0.53 | 1.34 | 0.33 | 1.14 |
| 4. Glucose 5 g/l and maltose 5 g/l | | 0.55 | 1.52 | 0.43 | 1.14 |
| 5. (4) containing sodium acetate 10 g/l | | 0.72 | 2.12 | 0.78 | 2.40 |
| 6. (5) excluding Tween 80® | | — | 2.00 | — | 0.10 |

This table indicates that the best result was obtained by using medium No. 5.

Example 3
Preparation of lactic acid:
A medium having the composition stated below was inoculated with *Lactobacillus italicus* TL-1 (FERM-BP 189; one platinum loop) for culturing at 30°C for 18 hours to obtain a cultured liquor containing lactic acid in an amount of 0.54%:
Composition of medium: yeast extract 12.5 g; peptone 12.5 g; glucose 11.0 g; maltose 15.0 g; $KH_2PO_4$ 0.25 g; $K_2HPO_4$ 0.025 g; $MgSO_4$. $7H_2O$ 0.1 g; $MnSO_4$ . $4H_2O$ 5 mg; $FeSO_4$ . $7H_2O$ 5 mg; Tween 80® 300 mg; L-cysteine HCl 300 mg; adenine 20 mg; guanine 20 mg; uracil 20 mg; sodium acetate. $3H_2O$ 10.0 g (made up to 1000 ml with water; pH being adjusted to 5.6 with hydrochloric acid).

**Claims**

1. A microorganism of the species *Lactobacillus italicus* having the following taxonomic characteristics:
(1) Gram-positive rods in a size of 0.8—1.0×2—7 μ;
(2) formation of spore: negative;
(3) motility: negative;
(4) anaerobic to facultative aerobic;
(5) growth temperature: 15—38°C, optimal 28—32°C;
(6) growth pH: 4.2—6.5, optimal 5.4—5.8;
(7) catalase: negative; and
(8) capable of assimilating glucose and maltose to produce DL-lactic acid, ethyl alcohol and carbon dioxide but not capable of assimilating arabinose, xylose, galactose, acetic acid, citric acid, gluconic acid or propionic acid as the sole source of carbon;
(9) DL-lactic acid and ethyl alcohol are produced from glucose or maltose at an approximate ratio of 1:0.4—0.6; and
(10) capable of growing in the absence of yeast; in or on a sterile medium.
2. A microorganism as claimed in claim 1 wherein glucose and maltose are the sole assimilable carbon sources.
3. A microorganism as claimed in claim 1 having no unsaturated fatty acid requirement for growth, said microorganism being designated *Lactobacillus italicus* TL-1 (FERM-BP 189).
4. A microorganism as claimed in claim 1 having an unsaturated fatty acid requirement for growth, said microorganism being designated *Lactobacillus italicus* SH 150 (FERM-BP 190).
5. A process for preparing a microorganism as claimed in claim 1 which comprises inoculating an agar medium with an aqueous suspension of a panettone dough, incubating the inoculated medium under anaerobic conditions to form colonies on said medium, isolating *Lactobacillus italicus* having the identifying characteristics defined in claim 1 and culturing the isolated strain(s) in or on a sterile medium.
6. A process as claimed in claim 5 wherein the isolation of *Lactobacillus italicus* comprises the isolation of *Lactobacillus italicus* TL-1 (FERM-BP 189) which has no unsaturated fatty acid requirement for growth and *Lactobacillus italicus* SH 150 (FERM-BP 190) which has an unsaturated fatty acid requirement for growth.

**0 113 215**

7. A process as claimed in claim 5 or claim 6 wherein the incubation is effected at a temperature of from 15—38°C for 1—2 days.

8. A process as claimed in any one of claims 5 to 7 wherein the incubation is effected at a pH of from 4.2—6.5.

9. A proces for the preparation of lactic acid which comprises culturing a microorganism as claimed in claim 1 in or on a medium therefor whereby to obtain a cultured liquor containing lactic acid.

10. A process as claimed in claim 9 wherein the lactic acid is isolated from the obtained cultured liquor.

## Patentansprüche

1. Mikroorganismus der Spezies Lactobacillus italicus mit folgenden taxonomischen Merkmalen:
   (1) gram-positive Stäbchen mit einer Größen von 0,8 bis 1,0×2 bis 7 μ;
   (2) Sporenbildung: negativ;
   (3) Beweglichkeit: negativ;
   (4) anaerob bis fakultativ aerob;
   (5) Wachstumstemperatur: 15—38°C, optimal 28—32°C;
   (6) Wachstums-pH: 4,2 bis 6,5, optimal 5,4 bis 5,8;
   (7) Katalase: negativ; und
   (8) Assimilierung von Glukose und Maltose zu DL-Milchsäure, Ethanol und Kohlendioxid, aber keine Assimilierung vo Arabinose, Xylose, Galactose, Essigsäure, Zitronensäure, Glukonsäure oder Propionsäure als einziger Kohlenstoffquelle,
   (9) DL-Milchsäure und Ethanol werden aus Glukose oder Maltose in einem Verhältnis von annähernd 1:0,4 bis 0,6 gebildet; und
   (10) Wachstum in Abwesenheit von Hefe, in oder auf einem sterilen Medium.

2. Mikroorganismus nach Anspruch 1, worin Glukose und Maltose die einzig assimilierbaren Kohlenstoffquellen bedeuten.

3. Mikroorganismus nach Anspruch 1, dessen Wachstum unabhängig von ungesättigten Fettsäuren erfolgt und der als Lactobacillus italicus TL-1 (FERM-BP 189) bezeichnet wird.

4. Mikroorganismus nach Anspruch 1, dessen Wachstum in Abhängigkeit von ungesättigten Fettsäuren erfolgt und der als Lactobacillus italicus SH 150 (FERM-BP 190) bezeichnet wird.

5. Verfahren zur Herstellung eines Mikroorganismus nach Anspruch 1 umfassend:
das Animpfen eines Agarmediums mit einer wäßrigen Suspension von Pannettone-Teig, Inkubation des angeimpften Mediums unter anaeroben Bedingungen zur Ausbildung von Kolonien auf dem Medium, Isolierung von Lactobacillus italicus mit den in Anspruch 1 bezeichneten Merkmalen und Kultivierung des isolierten Stammes (der isolierten Stämme) in oder auf einem sterilen Medium.

6. Verfahren nach Anspruch 5, worin die Isolierung von Lactobacillus italicus die Isolierung von Lactobacillus italicus TL-1 (FERM-BP 189), welcher für sein Wachstum keine ungesättigten Fettsäuren benötigt, und von Lactobacillus italicus SH 150 (FERM-BP 190), welcher zum Wachstum ungesättigte Fettsäuren benötigt, umfaßt.

7. Verfahren nach Anspruch 5 oder 6, worin die Inkubation bei einer Temperatur von 15 bis 38°C ein bis zwei Tage lang durchgeführt wird.

8. Verfahren nach einem der Ansprüche 5 bis 7, worin die Inkubation bei einem pH-Wert von 4,2 bis 6,5 durchgeführt wird.

9. Verfahren zur Herstellung von Milchsäure, welches die Kultivierung eines Mikroorganismus nach Anspruch 1 in oder auf einem entsprechenden Medium umfaßt, wobei man eine milchsäure-haltige Kulturflüssigkeit erhält.

10. Verfahren nach Anspruch 9, worin die Milchsäure aus der erhaltenen Kulturflüssigkeit isoliert wird.

## Revendications

1. Micro-organisme de l'espèce Lactobacillus italicus, présentant les caractéristiques taxonomiques suivantes:
   (1) bâtonnets Gram-positifs ayant des dimensions de 0,8—1,0×2—7 μm;
   (2) formation de spores: négatif;
   (3) mobilité: négatif;
   (4) anaérobie à aérobie facultatif;
   (5) température de croissance: 15—38°C, plage optimale 28—32°C;
   (6) pH de croissance: 4,2—6,5, plage optimale 5,4—5,8;
   (7) catalase: négatif; et
   (8) capable d'une assimilation du glucose et du maltose pour la production d'acide DL-lactique, d'alcool éthylique et d'anhydride carbonique, mais non capable d'une assimilation de l'arabinose, du xylose, du galactose, de l'acide acétique, de l'acide citrique, de l'acide gluconique ou de l'acide propionique comme seule source de carbone;
   (9) l'acide DL-lactique et l'alcool éthylique sont produits à partir de glucose ou de maltose en un rapport approximativement égal à 1:0,4—0,6; et

6

(10) capable d'une croissance en l'absence de levure; dans ou sur un milieu stérile.

2. Micro-organisme suivant la revendication 1, pour lequel le glucose et le maltose constituent les seules sources de carbone assimilable.

3. Micro-organisme suivant la revendication 1, n'ayant aucune exigence en acides gras insaturés pour sa croissance, ledit micro-organisme étant appelé *Lactobacillus italicus* TL-1 (FERM-BP 189).

4. Micro-organisme suivant la revendication 1, ayant une exigence en acides gras insaturés pour sa croissance, ledit micro-organisme étant appelé *Lactobacillus italicus* SH 150 (FERM-BP 190).

5. Procédé de préparation d'un micro-organisme suivant la revendication 1, qui consiste à ensemencer un milieu gélosé avec une suspension aqueuse d'une pâte à pain du type panettone, à faire incuber le milieu ensemencé dans des conditions anaérobies pour former des colonies sur ledit milieu, à isoler un *Lactobacillus italicus* ayant les caractéristiques d'identification définies suivant la revendication 1, et à cultiver la ou les souches isolées dans ou sur un milieu stérile.

6. Procédé suivant la revendication 5, dans lequel l'isolement de *Lactobacillus italicus* consiste à isoler *Lactobacillus italicus* TL-1 (FERM-BP 189) qui ne possède aucune exigence en acides gras insaturés pour sa croissance et *Lactobacillus italicus* SH 150 (FERM-BP 190) qui possède une exigence en acides gras insaturés pour sa croissance.

7. Procédé suivant la revendication 5 ou la revendication 6, dans lequel l'incubation est effectuée à une température de 15 à 38°C pendant 1 à 2 jours.

8. Procédé suivant l'une quelconque des revendications 5 à 7, dans lequel l'incubation est effectuée à un pH de 4,2 à 6,5.

9. Procédé de préparation d'acide lactique, qui consiste à cultiver un micro-organisme suivant la revendication 1 dans ou sur un milieu destiné à cet effet pour obtenir une liqueur de culture contenant de l'acide lactique.

10. Procédé suivant la revendication 9, dans lequel l'acide lactique est séparé de la liqueur de culture obtenue.